# FASCICULE DE BREVET EUROPEEN

(11) **EP 2 827 927 B1**
(45) Date de publication et mention de la délivrance du brevet: **27.06.2018**
(21) Numéro de dépôt: 13709218.5
(22) Date de dépôt: 15.03.2013
(51) Int. Cl.: A61M 15/00, A61M 16/20, F16K 15/14

(54) **VALVE DE TYPE EN BEC DE CANARD ET DISPOSITIF D'INHALATION COMPRENANT UNE TELLE VALVE**
ENTENSCHNABELVENTIL SOWIE INHALATIONSVORRICHTUNG MIT SOLCH EINEM VENTIL
DUCKBILL VALVE AND INHALATION DEVICE THEREFOR

(30) Priorité: 22.03.2012 FR 1252555
(43) Date de publication de la demande: 28.01.2015
(73) Titulaire: Protecsom, 50700 Valognes (FR)
(72) Inventeur: POREE, Thierry, F-50700 Valognes (FR)
(74) Mandataire: Cabinet Le Guen Maillet
(86) Numéro de dépôt international: PCT/EP2013/055321
(87) Numéro de publication internationale: WO 2013/139685

(56) Documents cités:
- EP-A2- 0 863 343
- FR-A1- 2 276 064
- US-A- 4 622 964
- US-A- 5 474 099
- US-B2- 6 557 549

## Description

La présente invention concerne une valve pour dispositif d'inhalation de substances médicamenteuses, ainsi qu'un dispositif d'inhalation comprenant une telle valve.

Des substances thérapeutiques actives peuvent être administrées par inhalation dans les poumons d'un patient. Ces substances sont par exemple administrées via des aérosols-doseurs dont un gaz propulseur génère un nuage de la substance active. De nombreux médicaments antiasthmatiques, destinés à soigner des pathologies telles que des affections broncho-pulmonaires, l'asthme, les bronchopathies, ou encore les bronchiolites, sont administrés à l'aide d'aérosols-doseurs.

L'utilisation d'une chambre d'inhalation est reconnue depuis longtemps pour faciliter et améliorer la médication, en particulier pour augmenter le dépôt des substances au niveau des bronches et pour réduire la vitesse du flux. En effet, en l'absence d'un tel dispositif d'inhalation, la coordination entre le déclenchement de l'aérosol-doseur et l'inspiration est primordiale. Or, cette coordination est difficile à réaliser pour le patient, notamment chez l'enfant.

Les dispositifs d'inhalation se présentent sous la forme d'une chambre délimitant un volume interne dans lequel l'aérosol est propulsé par l'intermédiaire d'une ouverture pratiquée à une extrémité de la chambre et sur laquelle est emboîté l'aérosol-doseur. A une autre extrémité de la chambre se trouve une autre ouverture en communication avec la bouche du patient par l'intermédiaire d'un moyen de liaison, généralement une partie tubulaire, tel qu'un embout que le patient peut prendre en bouche directement. Il est également possible de connecter un masque sur ce moyen de liaison, notamment dans le cas de dispositifs destinés aux jeunes enfants. La substance active est générée dans la chambre d'inhalation par pression sur l'aérosol-doseur. Puisque le patient inspire dans le moyen de liaison, la substance active est transportée jusque dans les bronches du patient par un flux sortant de la chambre qui est généré par le flux inspiratoire. Lors de l'expiration, le flux ne doit pas retourner à l'intérieur de la chambre. En pratique, une valve unidirectionnelle, dite valve inspiratoire, est prévue entre la chambre d'inhalation et la bouche du patient, au niveau de l'ouverture de la chambre débouchant dans le moyen de liaison, afin de gérer le flux de substances actives, en particulier, afin de permettre un flux sortant de la chambre à une vitesse adéquate et afin d'empêcher un flux entrant dans la chambre pendant l'expiration. Une ouverture pratiquée dans le moyen de liaison, en amont de la valve par rapport au patient, permet la sortie du flux expiratoire.

En ce qui concerne les valves unidirectionnelles, de nombreux dispositifs incluant une chambre d'inhalation utilisent des valves dites en bec de canard. Ce type de valve, bien connu de l'homme du métier, comporte une base en forme d'anneau définissant une ouverture centrale d'un diamètre déterminé. La valve est destinée à être traversée par un flux la pénétrant depuis cette ouverture centrale. Elle comporte encore deux parois obliques définissant respectivement une surface supérieure et une surface inférieure toutes deux planes, qui convergent l'une vers l'autre depuis la base dans une direction opposée à celle-ci jusqu'à ce que leurs bords libres se rejoignent et se touchent de sorte qu'elles forment un toit pointu. Les surfaces supérieure et inférieure forment un angle aigu entre elles. A la jonction des deux bords libres est réalisée une fente qui est transversale à l'axe central traversant la base annulaire. Les côtés de la valve, de part et d'autre des surfaces supérieure et inférieure sont bombés. Au repos, les bords libres des surfaces inférieure et supérieure sont joints. La fente de la valve est donc fermée. **Une telle valve est par exemple décrite dans le brevet** US 6 557 549**.** Lors de son utilisation dans un dispositif d'inhalation, la base de la valve est fixée dans un logement prévu dans le moyen de liaison entre la chambre et le patient.

Au cours de la phase inspiratoire du patient, sous la pression négative due à l'inspiration, les bords libres des surfaces inférieure et extérieure s'écartent l'un de l'autre en ouvrant la fente afin de permettre le passage d'un flux sortant de la chambre d'inhalation. A l'issue de la phase inspiratoire, les bords libres se rejoignent de nouveau et la fente se referme. Lors de la phase expiratoire suivante, la fente est maintenue close du fait de la pression expiratoire rendant ainsi impossible toute circulation de flux à travers la valve, notamment depuis l'intérieur de la chambre d'inhalation. Une telle valve est par exemple décrite dans la demande de brevet US 2007/0235028.

Ce type de valve est réalisé en une matière souple, telle que la silicone. L'épaisseur des parois et des surfaces planes doit être faible de manière à présenter une résistance faible à l'inspiration. Malheureusement ces valves présentent également un inconvénient non négligeable puisqu'elles sont susceptibles de se retourner facilement lorsque le souffle expiratoire du patient est trop élevé ou en cas de toux. Dans ce cas, les parois de valve passent au travers de la base annulaire en direction de l'intérieur de la chambre. Il en résulte un dysfonctionnement important avec fermeture de l'ouverture lors de la phase inspiratoire suivante et ouverture lors de la phase expiratoire. Si, au contraire, l'épaisseur des parois est augmentée, la résistance à l'inspiration augmente elle aussi.

Le but de la présente invention est de proposer une valve du type à bec de canard, notamment pour dispositif d'inhalation de substances médicamenteuses, qui présente à la fois une faible résistance à l'inspiration et une forte résistance à l'expiration et qui ne provoque pas de dysfonctionnement. En particulier, la valve selon l'invention ne doit pas se retourner lors de l'expiration.

A cet effet l'invention concerne une valve du type à bec de canard comportant une base en forme d'anneau définissant une ouverture centrale, une surface supérieure et une surface inférieure, toutes deux planes, qui convergent l'une vers l'autre depuis la base dans une direction opposée à celle-ci, caractérisée en ce que les surfaces supérieure et inférieure forment entre elles un angle d'au moins 60° et en ce que leurs bords libres délimitent une fente ouverte lorsque la valve est au repos.

Au sens de l'invention, on entend par « valve au repos », une valve qui n'est soumise à aucune pression telle que les pressions liées à l'inspiration ou à l'expiration lors de l'utilisation d'une valve dans un dispositif d'inhalation.

La valve selon l'invention présente ainsi une faible résistance à l'ouverture lorsqu'une pression est appliquée sur les faces internes des surfaces supérieure et inférieure du fait de son ouverture constante au repos. A l'inverse, la fente se ferme totalement lorsqu'une pression est exercée sur les faces externes des surfaces supérieure et inférieure. Lors de la fermeture de la fente, les bords libres des deux surfaces se touchent sur toute leur longueur. Cette fermeture est permise par l'angle formé entre ces deux surfaces.

Avantageusement, les bords libres des surfaces supérieure et inférieure présentent une découpe en courbe et concave. La découpe en courbe, ou curviligne, et concave des bords libres facilite la fermeture de la fente, c'est-à-dire le rapprochement jusqu'à la jonction des bords libres, lorsqu'une pression est exercée sur les faces externes des surfaces supérieure et inférieure.

Selon un mode de réalisation de l'invention, la tranche des bords libres est biseautée.

Selon une caractéristique de l'invention, l'ouverture centrale est de section circulaire ou ovale.

Selon un mode de réalisation de l'invention, la valve est en matériau élastomère, préférentiellement en silicone.

L'invention concerne encore un dispositif d'inhalation de substances médicamenteuses du type comportant une chambre d'inhalation et un moyen de liaison entre la chambre et le patient, tel qu'un embout buccal, ledit dispositif comprenant une valve selon l'invention et telle que définie précédemment, en particulier une valve du type à bec de canard comportant une base en forme d'anneau définissant une ouverture centrale, une surface supérieure et une surface inférieure, toutes deux planes, qui convergent l'une vers l'autre depuis la base dans une direction opposée à celle-ci, et dont les surfaces supérieure et inférieure forment entre elles un angle d'au moins 60° et en ce que leurs bords libres délimitent une fente ouverte lorsque la valve est au repos.

Selon un mode de réalisation de l'invention, le moyen de liaison entre la chambre et le patient comprend une ouverture équipée d'une fenêtre et débouchant dans une portion tubulaire en communication avec l'extérieur dudit dispositif et une valve apte à se déplacer entre deux positions, l'une dans laquelle elle se plaque contre la fenêtre, faisant barrière à tout passage de flux au travers de la fenêtre et l'autre dans laquelle elle s'éloigne de la fenêtre.

Selon un mode de réalisation de l'invention, la valve est plate et comporte une tige qui est insérée dans un logement aménagé dans l'axe central de la fenêtre, la tige étant libre de coulisser à l'intérieur du logement.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
La Fig. 1 représente une vue de profile d'un dispositif d'inhalation selon un mode de réalisation de l'invention,
La Fig. 2 représente une vue en perspective d'une valve et d'un col d'un dispositif d'inhalation selon un mode de réalisation de l'invention,
Les Figs. 3A et B représentent respectivement des vues de profile d'une valve selon un mode de réalisation de l'invention,
La Fig. 3C représente une vue du dessous d'une valve selon un mode de réalisation de l'invention,
Les Figs. 4A, B, C, représentent une vue du dessus d'une valve selon un mode de réalisation de l'invention, au repos (Fig. 4A), lors d'une phase d'inhalation en fonctionnement dans un dispositif d'inhalation (Fig. 4 B) et lors d'une phase d'expiration en fonctionnement dans un dispositif d'inhalation (Fig. 4 C),
La Fig. 5 représente une vue explosée d'une valve d'expiration selon un mode de réalisation de l'invention,
La Fig. 6A et B représente une vue en coupe selon l'axe A-A' d'une valve d'expiration en fonctionnement selon un mode de réalisation de l'invention,
Les Figs. 7 et 8 représentent le fonctionnement d'une valve de type à bec de canard et d'une valve d'expiration selon un mode de réalisation de l'invention lors d'une phase d'inspiration (Fig. 7) et lors d'une phase d'expiration (Fig. 8).

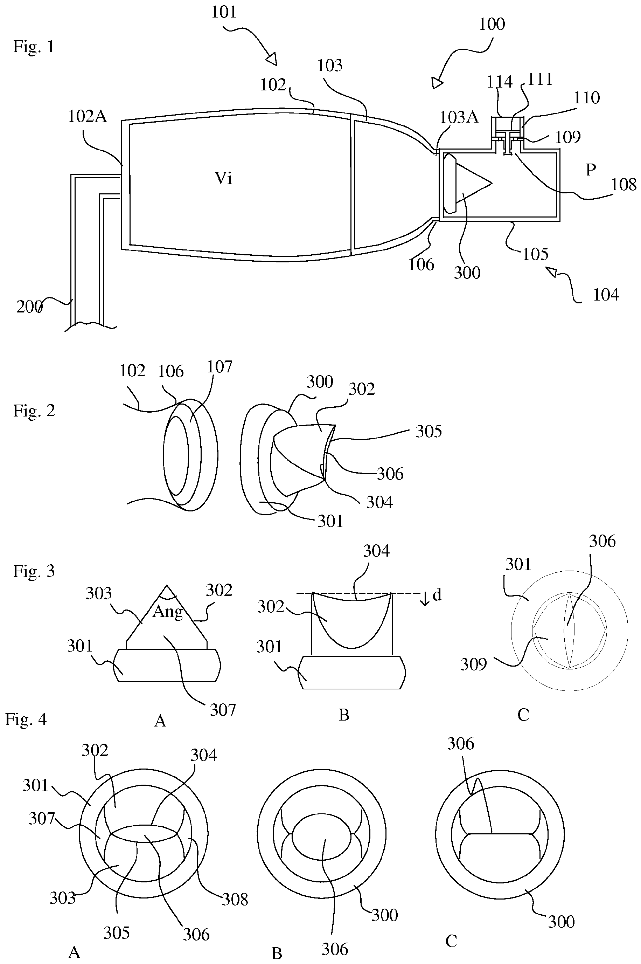

Sur la Fig.1 est représenté un dispositif d'inhalation 100, notamment pour l'administration des traitements inhalés destinés aux soins d'affections broncho-pulmonaires, comportant une chambre d'inhalation 101 composée dans ce mode de réalisation de deux parties sensiblement tubulaires 102, 103 l'une emboîtée dans l'autre. La chambre d'inhalation peut bien entendu se présenter sous une autre forme, telle que deux parties tronconiques ou une seule partie tubulaire. Une première extrémité de la chambre 102A est abouchée à un aérosol doseur 200 contenant un principe actif et une seconde extrémité opposée 103A est abouchée à un moyen de liaison 104 entre la chambre 101 et le patient P, précisément un embout buccal 105. Le moyen de liaison 105 peut être un masque facial. Le moyen de liaison 105 s'emboîte sur un col 106 que comporte l'extrémité 103A de la chambre. Il est facilement démontable.

Une valve de type à bec de canard 300 est prévue en amont du patient P par rapport au volume interne Vi de la chambre 101. Cette valve 300 est une valve unidirectionnelle et inspiratoire autorisant le passage d'un flux transportant les particules médicamenteuses depuis l'intérieur de la chambre 101 en direction du patient lors de la phase inspiratoire du patient P.

Un logement 107 pour la valve du type à bec de canard 300 est aménagé dans le col 106 du dispositif 100. Il s'agit, par exemple, d'une gorge, d'une rainure ou d'un méplat (Fig. 2).

La valve de type à bec de canard 300 selon l'invention est illustrée plus en détail en lien avec les Figs 2, 3 et 4. Elle comporte une base 301, en forme d'anneau ou de collerette, définissant une ouverture centrale 309 d'un diamètre déterminé (Fig. 3 C). La base 301 est prévue pour siéger dans le logement 107 du dispositif 100 (Fig. 2).

La valve 300 présente deux parois obliques formant une surface supérieure 302 et une surface inférieure 303, toutes deux planes. Les côtés 307, 308 de part et d'autre des surfaces supérieure et inférieure 302, 303 sont bombés.

Les surfaces supérieure 302 et inférieure 303 convergent l'une vers l'autre depuis la base 301 dans une direction opposée à celle-ci et forment entre elles un angle Ang d'au moins 60°. Lorsqu'elle est vue de profil, avec un côté 307 ou 308 faisant face, la valve 300 a la forme d'un bec de canard ou encore d'un toit (Fig. 3A).

Les surfaces 302, 303 présentent une forme de semi ellipse.

Les bords libres 304, 305 des surfaces supérieure et inférieure 302, 303 sont courbes et concaves. La concavité est tournée vers la base 301 (Fig. 3B, flèche d) et est faiblement prononcée, de l'ordre de 0,2 à 1 mm.

Du fait de leur découpe en courbe et concave, les bords libres 304, 305 ne se touchent pas sur toute leur longueur et délimitent une fente 306 qui est transversale à l'axe central traversant la base annulaire 301. La fente 306 est ouverte de manière visible au repos, c'est à dire en dehors de toute utilisation, comme cela est illustré aux Figs. 3C et 4A.

La valve 300 est réalisée par moulage en matériau élastomère, préférentiellement en silicone. La découpe des bords en arrondi est réalisée après démoulage.

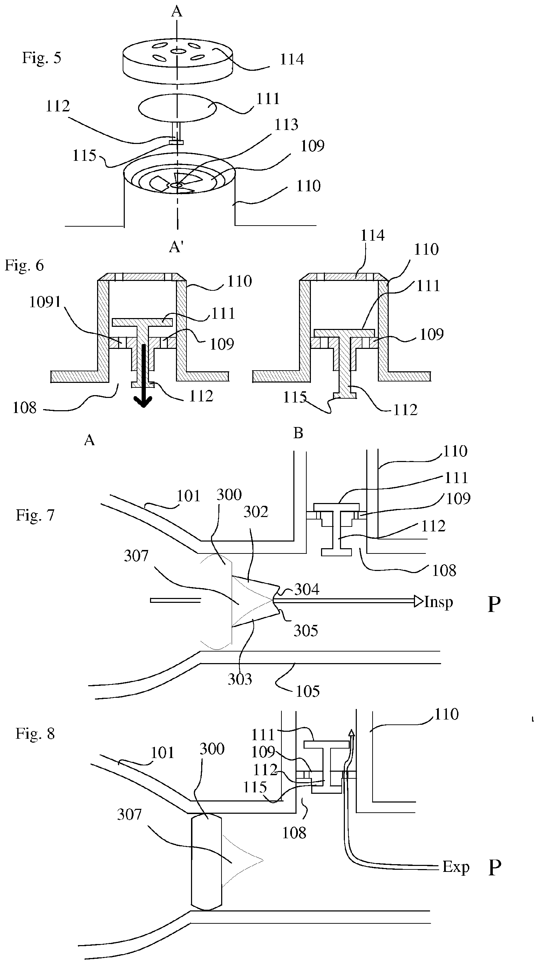

Comme cela est visible sur la Fig.1, le moyen de liaison 105 entre la chambre 101 et le patient P comprend encore une ouverture 108 prévue pour la sortie du flux expiratoire du patient lorsque celui-ci expire dans le dispositif 100. L'ouverture 108 est équipée d'une fenêtre 109 comportant une ou plusieurs ouvertures et débouche dans une portion tubulaire 110 elle même en communication avec l'extérieur du dispositif 100. Le détail de la portion tubulaire 110 est illustré à la Fig. 5.

Une valve plate 111, circulaire et souple est prévue dans la portion tubulaire 110 pour obturer les ouvertures de la fenêtre 109 lors de la phase d'inspiration. La valve plate 111 comporte une tige centrale 112 qui est insérée dans un logement 113 aménagé dans l'axe central de la fenêtre 109 (Fig. 5). La portion tubulaire 110 est couverte par un chapeau 114 comportant des ouvertures. Le chapeau 114 est simplement emboîté sur la portion 110 et se trouve de ce fait, facilement démontable.

La tige 112 est libre de coulisser dans l'axe du logement 113. La valve 111 peut ainsi se déplacer entre deux positions, une position dite de fermeture dans laquelle elle plaque sa portion plate contre la fenêtre 109 d'accès à l'embout buccal 105 (Fig. 6A), faisant ainsi barrière à tout passage de flux au travers des ouvertures 1091 de la fenêtre 109 et une autre position, dite d'ouverture, dans laquelle elle s'éloigne de la fenêtre 109 de manière à laisser libre le passage d'un flux au travers de la fenêtre (Fig. 6 B). La tige 112 comporte un pied 115 faisant butée contre le logement 113. Le pied 115 permet d'éviter que la tige quitte le logement 113 lorsqu'elle adopte la position d'ouverture.

Les Figs. 7 et 8 illustrent le fonctionnement de la valve 300 ainsi que la valve d'expiration 111, dans le dispositif d'inhalation 100.

Lorsque le patient P inspire, il se crée une dépression dans le moyen de liaison 105. La pression est forte sur les faces internes des surfaces supérieure et inférieure 302, 303. Les bords libres 304, 305 s'éloignent l'un de l'autre et la fente 306 de la valve 300 s'ouvre davantage qu'au repos. La valve 300 présente une faible résistance à l'inspiration du fait de son ouverture constante au repos. Le flux inspiratoire, représenté par la flèche, qui transporte la substance médicamenteuse, peut traverser la valve 300 depuis la chambre 101 en direction du patient P (fig. 7).

Pendant que la valve 300 s'ouvre pour permettre le passage du flux inspiratoire, la valve 111 se place dans une position dans laquelle elle ferme le passage au travers de la fenêtre 109 en se plaquant contre elle (Fig. 7).

A l'inverse, lors de la phase suivante d'expiration du patient dans le moyen de liaison 105, une pression est exercée sur les faces externes des surfaces supérieure et inférieure 302, 303. La fente 306 se ferme totalement par rapprochement et jointure des bords libres 304, 305. Les bords libres 304, 305 se touchent cette fois ci sur toute leur longueur (Fig. 8). Cette fermeture est favorisée par l'angle Ang supérieur ou égal à 60° formé entre les surfaces inférieure et supérieure 302, 303. La découpe en courbe et concave des bords libres 304, 305 facilite également la fermeture de la fente 306.

Pendant que la valve 300 se ferme, la tige 112 de la valve 111 coulisse dans l'axe 113 et la valve 11 s'éloigne de la fenêtre 109 en se plaçant dans une position dans laquelle elle permet le passage du flux expiratoire (voir flèche) au travers de la fenêtre 109 en direction de l'extérieur. Le pied 115 stoppe la course de la tige 112 lorsqu'il vient buter contre les parois du logement 113 (Fig. 8).

La valve 300 et la valve 111 sont démontables et lavables.

## Revendications

1. Valve du type à bec de canard (300) comportant une base (301) en forme d'anneau définissant une ouverture centrale (309), une surface supérieure (302) et une surface inférieure (303), toutes deux planes, qui convergent l'une vers l'autre depuis la base (301) dans une direction opposée à celle-ci, **caractérisée en ce que** les surfaces supérieure et inférieure (302, 303) forment entre elles un angle (Ang) d'au moins 60° et **en ce que** leurs bords libres (304, 305) délimitent une fente (306) ouverte lorsque la valve est au repos.

2. Valve (300) selon la revendication 1, **caractérisée en ce que** les bords libres (304, 305) des surfaces supérieure et inférieure (302, 303) présentent une découpe en courbe et concave.

3. Valve (300) selon la revendication 1 ou 2, **caractérisée en ce que** la tranche des bords libres (304, 305) est biseautée.

4. Valve (300) selon l'une des revendications précédentes, **caractérisée en ce que** l'ouverture centrale 309 est de section circulaire ou ovale.

5. Valve (300) selon l'une des revendications précédentes, **caractérisée en ce qu'**elle est en matériau élastomère, préférentiellement en silicone.

6. Dispositif d'inhalation (100) de substances médicamenteuses du type comportant une chambre d'inhalation (101) et un moyen de liaison (105) entre la chambre (101) et un patient (P), tel qu'un embout buccal (104), **caractérisé en ce qu'**il comporte une valve (300) selon l'une des revendications 1 à 5.

7. Dispositif d'inhalation (100) selon la revendication 6, **caractérisé en ce que** le moyen de liaison (105) entre la chambre et le patient comprend une ouverture (108) au niveau de laquelle est montée une fenêtre (109) comportant des ouvertures (1091) et débouchant dans une portion tubulaire (110) en communication avec l'extérieur dudit dispositif (100) et une valve (111) apte à se déplacer entre deux positions, l'une dans laquelle elle se plaque contre la fenêtre 109, faisant barrière à tout passage de flux au travers de la fenêtre (109) et l'autre dans laquelle elle s'éloigne de la fenêtre (109).

8. Dispositif d'inhalation (100) selon la revendication 6 ou 7, **caractérisé en ce que** la valve (111) est plate et comporte une tige (112) qui est insérée dans un logement (113) aménagé dans l'axe central de la fenêtre (109), ladite tige (112) étant libre de coulisser à l'intérieur dudit logement (113).

## Patentansprüche

1. Entenschnabelventil (300), welches einen Sockel (301) in Form eines Ringes, der eine zentrale Öffnung (309) definiert, eine obere Fläche (302) und eine untere Fläche (303), die beide eben sind und die vom Sockel (301) aus in einer zu ihm entgegengesetzten Richtung aufeinander zulaufen, umfasst, **dadurch gekennzeichnet, dass** die obere und die untere Fläche (302, 303) miteinander einen Winkel (Ang) von mindestens 60° bilden, und dadurch, dass ihre freien Ränder (304, 305) einen Spalt (306) begrenzen, der offen ist, wenn sich das Ventil im Ruhezustand befindet.

2. Ventil (300) nach Anspruch 1, **dadurch gekennzeichnet, dass** die freien Ränder (304, 305) der oberen und der unteren Fläche (302, 303) einen gekrümmten und konkaven Ausschnitt aufweisen.

3. Ventil (300) nach Anspruch 1 oder 2, **dadurch gekennzeichnet, dass** die Schnittfläche der freien Ränder (304, 305) abgeschrägt ist.

4. Ventil (300) nach einem der vorhergehenden Ansprüche, **dadurch gekennzeichnet, dass** die zentrale Öffnung 309 einen kreisförmigen oder ovalen Querschnitt hat.

5. Ventil (300) nach einem der vorhergehenden Ansprüche, **dadurch gekennzeichnet, dass** es aus elastomerem Material hergestellt ist, vorzugsweise aus Silikon.

6. Inhalationsvorrichtung (100) für Arzneistoffe, des Typs, welcher eine Inhalationskammer (101) und ein Mittel zur Verbindung (105) zwischen der Kammer (101) und einem Patienten (P), wie etwa ein Mundstück (104), aufweist, **dadurch gekennzeichnet, dass** sie ein Ventil (300) nach einem der Ansprüche 1 bis 5 aufweist.

7. Inhalationsvorrichtung (100) nach Anspruch 6, **dadurch gekennzeichnet, dass** das Mittel zur Verbindung (105) zwischen der Kammer und dem Patienten eine Öffnung (108) umfasst, an der ein Öffnungen (1091) aufweisendes Fenster (109) angebracht ist und die in einen rohrförmigen Abschnitt (110) mündet, der mit der Außenseite der Vorrichtung (100) in Verbindung steht, und ein Ventil (111), das sich zwischen zwei Positionen verlagern kann, einer, in der es an das Fenster (109) angedrückt wird und dabei eine Sperre für jeden Durchfluss durch das Fenster (109) bildet, und einer anderen, in der es sich von dem Fenster (109) entfernt.

8. Inhalationsvorrichtung (100) nach Anspruch 6 oder 7, **dadurch gekennzeichnet, dass** das Ventil (111) flach ist und eine Stange (112) aufweist, welche in eine Aufnahme (113) eingefügt ist, die in der zentralen Achse des Fensters (109) angeordnet ist, wobei die Stange (112) im Inneren der Aufnahme (113) frei gleiten kann.

## Claims

1. Valve (300) of the duckbill type comprising a base (301) in the form of a ring defining a central opening (309), an upper surface (302) and a lower surface (303), both planar, which converge towards one another from the base (301) in an opposite direction thereto, **characterized in that** the upper and lower surfaces (302, 303) form between them an angle (Ang) of at least 60°, and **in that** their free edges (304, 305) delimit a slot (306) which is open when the valve is at rest.

2. Valve (300) according to Claim 1, **characterized in that** the free edges (304, 305) of the upper and lower surfaces (302, 303) have a curved and concave cutout.

3. Valve (300) according to Claim 1 or 2, **characterized in that** the side part of the free edges (304, 305) is bevelled.

4. Valve (300) according to one of the preceding claims, **characterized in that** the central opening 309 is of circular or oval cross section.

5. Valve (300) according to one of the preceding claims, **characterized in that** it is made of elastomer material, preferably of silicone.

6. Device (100) for inhalation of medicinal substances of the type comprising an inhalation chamber (101) and a means (105) of connection between the chamber (101) and a patient (P), such as a mouthpiece (104), **characterized in that** it comprises a valve (300) according to one of Claims 1 to 5.

7. Inhalation device (100) according to Claim 6, **characterized in that** the means (105) of connection between the chamber and the patient comprises an opening (108) at which there is mounted a window (109) comprising openings (1091) and opening into a tubular portion (110) in communication with the outside of the said device (100) and a valve (111) able to move between two positions, one in which it is pressed against the window 109, forming a barrier to any passage of flow between the window (109), and the other in which it moves away from the window (109).

8. Inhalation device (100) according to Claim 6 or 7, **characterized in that** the valve (111) is flat and comprises a stem (112) which is inserted into a housing (113) formed in the central axis of the window (109), the said stem (112) being free to slide within the said housing (113).
